(19) Europäisches Patentamt / European Patent Office / Office européen des brevets

(11) **EP 4 039 293 A1**

(12) **EUROPEAN PATENT APPLICATION**

(43) Date of publication:
**10.08.2022 Bulletin 2022/32**

(21) Application number: **22155269.8**

(22) Date of filing: **04.02.2022**

(51) International Patent Classification (IPC):
*A61M 5/142* (2006.01)

(52) Cooperative Patent Classification (CPC):
**A61M 5/14248;** A61M 2205/3561;
A61M 2205/3584; A61M 2205/52; A61M 2230/201

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.02.2021 US 202163146009 P**

(71) Applicant: **Insulet Corporation
Acton MA 01720 (US)**

(72) Inventors:
• ZHENG, Yibin
  Harland, 53029 (US)
• LEE, Joon Bok
  Acton, 01720 (US)
• O'CONNOR, Jason
  Acton, 01720 (US)

(74) Representative: **Peterreins Schley
Patent- und Rechtsanwälte PartG mbB
Hermann-Sack-Strasse 3
80331 München (DE)**

(54) **TECHNIQUES AND DEVICES FOR ADAPTATION OF MAXIMUM DRUG DELIVERY LIMITS**

(57) Disclosed are techniques, a system and devices that enable the setting of an upper boundary constraint that may be a multiple of a total daily dosage setting for a liquid drug being administered to a user to control a condition, such as type 1 or type 2 diabetes mellitus. An automatic drug delivery algorithm may be configured to obtain a glucose control metric. A controller executing the automatic drug delivery algorithm may ascertain, based on the glucose control metric, an upper boundary constraint for the liquid drug that limits an amount of a dose of the liquid drug that may be delivered by the automatic drug delivery system or components thereof.

**FIG. 3**

EP 4 039 293 A1

**Description**

BACKGROUND

The maximum insulin delivery limits for AID systems are typically established by an assessment of the maximum variations in the user's insulin needs over time. The insulin needs of Type 1 diabetes-mellitus (T1DM) users may vary based on the user's lifestyle, stress, illness, and time of day, and typical AID insulin delivery limits are approximately 3-4 times the user's basal needs.

The insulin needs of persons with Type 2 diabetes-mellitus (T2DM) may vary by a significantly greater margin than persons with T1DM, due to the additional factor of disease progression. T2DM patients may require much larger amounts of insulin as their body increasingly loses ability to use endogenous insulin or may start requiring no insulin as their disease progression improves. Whereas the disease progression of T1DM patients remains relatively constant (once the T1DM patient loses the capability to produce endogenous insulin, they do not regain it).

Due to the differences in the types of diabetes, persons with T2DM do not respond to insulin as quickly or efficiently as a person with T1DM, and as the T2DM disease progresses, the persons with T2DM do not produce enough insulin. Simply stated, T2DM diabetics are resistant to insulin, while a person with T1DM does not produce insulin. As a result, a person with T2DM may need a greater amount of insulin than a person with T1DM.

Therefore, it would be helpful if there was a way to adjust delivery limits of automated devices that deliver drugs and thereby deliver a greater amount of a drug, such as insulin, for T2DM patients as compared to T1DM patients to account for the variabilities of T2DM.

BRIEF SUMMARY

In one aspect, a wearable drug delivery device including a controller, a memory, a container containing a liquid drug, and a pump mechanism is disclosed. The pump mechanism may be coupled to the controller and coupled to the pump mechanism. The pump mechanism may be configured to expel the liquid drug from the container in response to control signals from the controller. The memory may store an automatic drug delivery algorithm, the automatic drug delivery algorithm, when executed by the controller, may configure the controller to receive an indication of a type of diabetes that will be controlled by the controller and obtain a glucose control metric. The controller may ascertain, based on the indication of the type of diabetes and the glucose control metric, an upper boundary constraint for the liquid drug. The upper boundary constraint may be a multiple of a total daily dosage setting for the liquid drug. An amount of a dose of the liquid drug to be delivered may be determined based on the upper boundary constraint. A control signal may be generated based on the determined amount of the dose of the liquid drug. The controller may apply the control signal to the pump mechanism to expel the dose of the liquid drug from the container.

In another aspect, a non-transitory computer readable medium embodied with programming code is disclosed. The programming code may cause a processor when executing the programming code to receive an indication of a type of diabetes that will be controlled by the processor. A glucose control metric may be obtained. The processor may ascertain, based on the indication of the type of diabetes and the glucose control metric, an upper boundary constraint for the liquid drug. The upper boundary constraint is a multiple of a total daily dosage setting for the liquid drug. An amount of a dose of the liquid drug to be delivered based on the upper boundary constraint may be determined. A control signal may be generated based on the determined amount of the dose of the liquid drug. The control signal may be applied to the pump mechanism to expel the dose of the liquid drug from the container.

In a further aspect, a drug delivery system that includes a processor, a communication interface, and a memory is disclosed. The drug delivery system also includes a communication interface coupled to the processor. The drug delivery system also includes a memory storing an automatic drug delivery algorithm, where the automatic drug delivery algorithm, when executed by the processor, configures the processor to determine an average blood glucose measurement value over a period of time. The processor may be configured to determine an estimated glucose control metric using the average blood glucose measurement value. Based on the estimated glucose control metric, an upper boundary constraint for the liquid drug may be calculated. An amount of a dose of the liquid drug to be delivered may be determined based on the calculated upper boundary constraint. The processor may be configured to generate a control signal based on the determined amount of the dose of the liquid drug and output the control signal to cause a pump mechanism to expel the dose of the liquid drug from a container.

In yet another aspect, a non-transitory computer-readable storage medium is disclosed. The non-transitory computer-readable storage medium includes instructions that when executed by a processor, cause the processor to determine an average blood glucose measurement value over a period of time. An estimated glucose control metric may be estimated using the average blood glucose measurement value. The processor may calculate, based on the estimated glucose control metric, an upper boundary constraint for the liquid drug, and determine a time for delivery of the liquid drug to the user. Prior to the determined time, an amount of a dose of the liquid drug to be delivered may be determined based on the calculated upper boundary constraint. A control signal may be generated based on the determined amount of the dose of the liquid drug and the control signal may be output to cause a pump mechanism to expel the dose of the liquid drug from a container.

BRIEF DESCRIPTION OF THE DRAWINGS

To easily identify the discussion of any particular element or act, the most significant digit or digits in a reference number refer to the figure number in which that element is first introduced.

FIG. 1 illustrates a process 100 in accordance with one example.

FIG. 2 illustrates a process 200 in accordance with another example.

FIG. 3 illustrates a system example suitable for implementing the processes described herein.

FIG. 4 illustrates a graphic usable in describing the subject matter of the process examples of FIGs. 1 and 2.

FIG. 5 illustrates an example of a data structure suitable for use in the processes described herein.

DETAILED DESCRIPTION

The following examples describe how an upper boundary of a total daily limit of a drug dosage is increased to allow more of the drug to be delivered to a user. The benefits to increasing the upper boundary may be to permit automatic drug delivery systems to deliver more appropriate amounts of the drug to users whose illness or physical condition may cause wider fluctuations in physical attributes that are controlled by the drug. The more appropriate amount of the drug may reduce the fluctuation in the user's physical attributes, such as blood glucose. The following examples disclose a variety of techniques and devices that may determine a maximum delivery limit that is equivalent to the relaxed upper boundary and that further deliver a drug dosage based on the maximum delivery limit to a user. The examples describe long-range determinations of the maximum delivery limits and short-range determinations of the maximum delivery limits.

The upper boundary number, or maximum delivery limit, may be set for the drug delivery device, such as an insulin pump or the like, to limit the operating range of the automatic drug delivery device with respect to a maximum daily amount of a drug that may be delivered via basal delivery. Basal delivery may also be referred to as "a basal dose," "a basal dosage," or "a background dosage." For example, if the drug is insulin, the automatic drug delivery device may deliver over the course of a day, a basal dosage of insulin (e.g., once an hour for 24 hours, every 5 or 10 minutes over 24 hours, or the like). The maximum delivery limit may be a total daily limit, such as, in the case of insulin, total daily insulin (TDI), of the basal dosages of insulin delivered to the user by the automatic drug delivery device.

In an example, the maximum delivery limit is used by an automatic drug delivery algorithm or application in the calculation of an amount of insulin to be delivered to a user over a period of time. An automatic drug delivery algorithm or application may also be referred to as "a medication delivery algorithm or application" and the terms may be used interchangeably. As a result, by increasing the maximum delivery limit, the automatic drug delivery algorithm may expand the amounts of insulin that may be delivered to the user to improve management of the user's blood glucose and diabetes overall. For example, the automatic drug delivery algorithm or application may generate wider recommendations for insulin doses to be delivered over the course of a period of time, such as a day, by a multiplier of total daily insulin delivered via basal doses.

Since users with T2DM have a wider variability of their insulin needs and are less susceptible to hypoglycemia due to their bodies ability to still generate insulin, an improvement of the described examples it the expansion of the upper boundary which enables the automatic drug delivery algorithm or application to more easily address the fluctuations in the T2DM user's insulin via the automatic drug delivery algorithm or application.

Systems utilizing an automatic drug delivery algorithm or application may be configured to modulate basal dosages up to the expanded upper boundary, which allows the automatic drug delivery algorithm or application to command larger basal dosages, more frequent basal dosages, or both to be delivered.

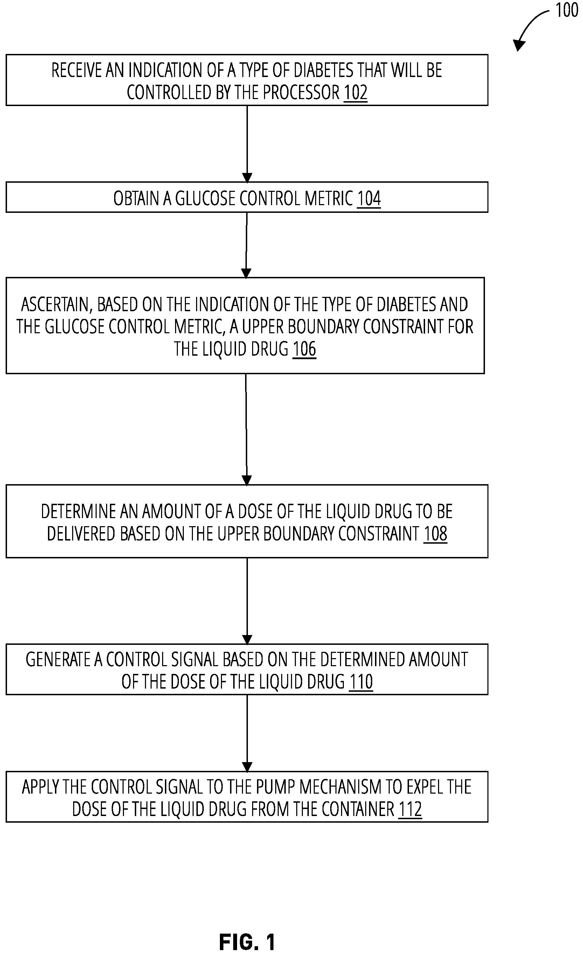

FIG. 1 illustrates a process 100 that implements an example of the disclosed subject matter. In block 102, process 100 receives an indication of a type of diabetes to be controlled by the processor. In the example, the automatic drug delivery algorithm or application may be configured to execute different processes and apply different settings depending upon whether the indication is of the type of diabetes being either T1DM or T2DM for the reasons discussed above. The examples discussed herein are directed to managing upper boundary constraints for users diagnosed with T2DM. In the step of block 102, the controller may receive the indication of the type of diabetes at an initial set up of a management device, a smart accessory device, or a wearable drug delivery device (each described in more detail with reference to a later system example).

In a further example, the controller may at block 102 be further configured to receive an indication of a comorbidity of a user in addition to the indication of the type of diabetes. In the example, a user may have a comorbidity that may be obesity, advanced age (e.g., greater than 65 years of age), high blood pressure, heart disease, asthma or another respiratory disease, or the like. Using the indication of the comorbidity either with the indication of the type of diabetes, the controller may be configured to further adjust the upper boundary constraint for the liquid drug. Alternatively, the controller may be configured to further adjust the upper boundary constraint for the liquid drug based on the comorbidity after the upper boundary constraint has been determined based on the indication of the type of diabetes. In the example of FIG. 1, the controller may be further configured to use the indication of the type of diabetes and indication of the comorbidity.

In block 104, process 100 obtains a glucose control metric. The glucose control metric may be represented as a percentage of red blood cells that have sugar-coated hemoglobin over a period of time. Examples of the glucose control metric include but are not limited to glycated hemoglobin, HbAlc from laboratory test results, an estimated HbAlc based on blood glucose measurement values, or the like. In an operational example, the controller may calculate the glucose control metric as an estimate of HbAlc % based on blood glucose measurement values received via a communication interface. Alternatively, a communication interface (described in another example) may receive an indication of the glucose control metric via a wireless signal (from an external device or cloud-based service) or a haptic input (e.g., via a push button, a touch screen or the like), and the indication is forwarded to the controller.

In block 106, process 100 ascertains an upper boundary constraint for the liquid drug based on the indication of the type of diabetes and the glucose control metric (as well as a comorbidity, if provided). The upper boundary constraint may be a multiple of a total daily dosage setting for the liquid drug. In an example, the controller based on the indication of the type of diabetes, may evaluate the glucose control metric to determine a setting for the multiple of the total daily dosage setting. Upon determining the setting for the multiple of the total daily dosage setting, the controller may store the glucose control metric, the determined multiple, and the upper boundary constraint in a memory.

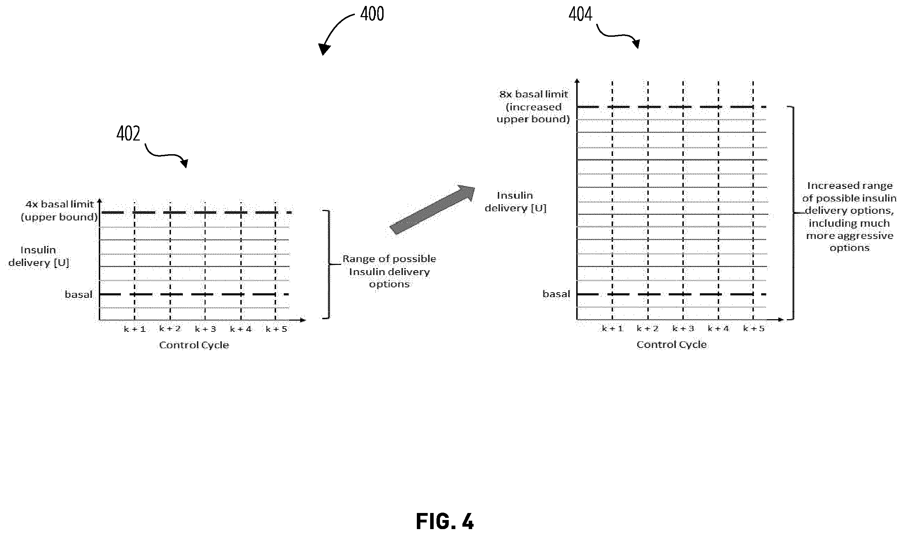

For example, in response to the indication of the type of diabetes indicating Type 1 diabetes mellitus, the controller may set the multiple of the total daily dosage setting to a fixed clinical multiple setting. As shown in FIG. 4, the controller, when evaluating the indication of the type of diabetes is Type 1 diabetes, may select in memory a table that indicates an upper boundary constraint, or limit (shown as fixed clinical multiple setting 402), which in this example is set at four times (4x) the basal limit for a dosage. Of course, multiples other than 4x may be used, such as 3.5x, 5x, 6x or the like. The fixed clinical multiple setting 402 may be determined from a look up table that has a range of glucose control metrics. Once the controller has set the multiple, the controller may store the glucose control metric, the fixed clinical multiple setting, and the upper boundary constraint in the memory.

Alternatively, in response to the indication of the type of diabetes indicating Type 2 diabetes mellitus, the glucose control metric may be evaluated to determine the multiple of the total daily dosage setting. As shown in FIG. 4 are upper bound examples 400, the controller, when evaluating the indication of the type of diabetes is Type 2 diabetes, may select in memory a table that indicates a maximum upper boundary constraint, or limit (shown as increased upper bound 404), at eight times (8x) the basal limit for a dosage. During the evaluation in response to the indication of the type of diabetes indicating Type 2 diabetes mellitus, the controller may select a multiple from a list of multiples of the total daily dosage setting stored in a memory. For example, the selected multiple may correspond to the glucose control metric found in the list. In the example, the multiple may increase based on an increase in a range of the glucose control metric. The controller may be further configured to, in response to the indication of the type of diabetes being for Type 2 diabetes mellitus, select a multiple from a list of multiples of the total daily dosage setting. In the example, the selected multiple may correspond to a glucose control metric threshold. In a further example, the multiple may increase based on a specific glucose control metric called HbA1c. Examples of HbAlc thresholds may be 7.0%, 7.5%, 8.0%, 8.5% and the like. The tolerance of HbAlc threshold may be 0.5%-1%, for example.

The glucose control metric, the determined multiple, and the upper boundary constraint may be stored in the memory. Alternatively, the glucose control metric and the upper boundary, or just the upper boundary may be stored in the memory.

Additionally, the process 100 may determine a time for delivery of the liquid drug to the user. The controller may determine a dosage of the drug that is to be delivered at a specified time based on the multiple selected, for example based on based on a specific glucose control metric. The specified time may be based on the multiple selected.

In block 108, process 100 may determine an amount of a dose of the liquid drug to be delivered based on the upper boundary constraint. The adjusted upper boundary constraint may be used to determine the amount of the dose of the liquid drug to be delivered based on the upper boundary constraint.

In block 110, process 100 generates a control signal based on the determined amount of the dose of the liquid drug. In block 112, process 100 applies the control signal to the pump mechanism to expel the dose of the liquid drug from the container.

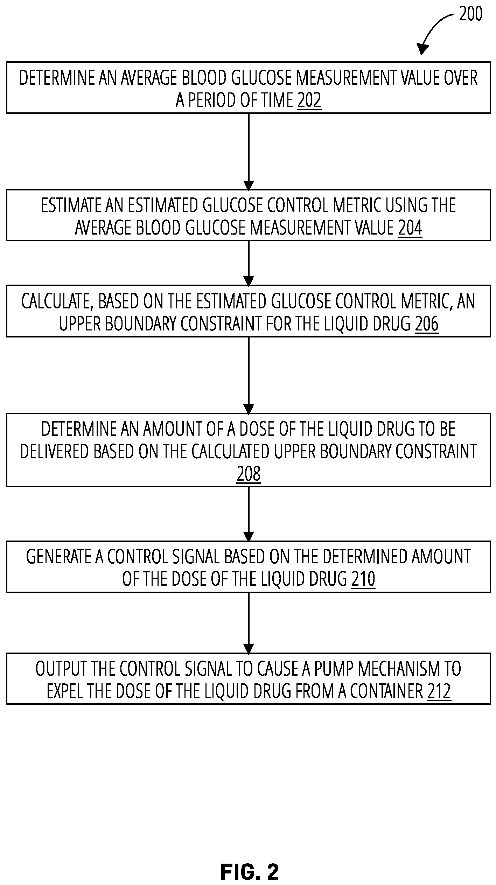

FIG. 2 illustrates another example of a process that estimates a blood glucose control metric for use in determining an upper boundary constraint.

In block 202, process 200 determines an average blood glucose measurement value over a period of time. In an example, a controller may retrieve the blood glucose history from a memory coupled to the controller. A number of blood glucose measurements may be identified over the period of time in the blood glucose history. The average blood glucose measurement value may be calculated using the identified number of blood glucose measurements.

In block 204, process 200 estimates an estimated glucose control metric using the average blood glucose measurement value.

In block 206, process 200 calculates, based on the estimated glucose control metric, an upper boundary constraint for the liquid drug. The calculated upper boundary constraint may be calculated based on a previous upper boundary constraint and an interim upper boundary constraint for the maximum drug delivery limit for the liquid drug. The previous upper boundary constraint and an interim upper boundary constraint may be obtained by a controller from a memory coupled to the controller.

In another example at block 206, the upper boundary constraint may be calculated by determining a number of days covered by the period of time, such as 14 days. A proportion of the number of days may be attributed to the previous upper boundary constraint and a remainder of the proportion may be attributed to the interim upper boundary constraint. A controller executing a medication delivery application may multiply the previous upper boundary constraint by the proportion of the number of days to be attributed to the previous upper boundary constraint to obtain a first result. The controller may multiply the interim upper boundary constraint by the remainder of the proportion attributed to the interim upper boundary constraint to obtain a second result. The first result and the second result may be summed together, and the sum may be stored in a memory as the calculated upper boundary constraint.

For example, the above discussion may implement the following:

$$UB_{new} = \left(1 - X \cdot N_{days}\right)UB_{h-1} + X \cdot N_{days}UB_h$$

$$UB_h = f\left(F_{adj}\right)$$

where $N_{days}$ is the number of days of glucose history available, $UB_h$ and $UB_{h-1}$ are current and previous upper bounds, $CGM(n)$ are CGM values in the current history segment, and $X$, such as 0.2, is a tunable factor to determine how fast the system adapts to changes in a user's upper boundary settings. The function $f(F_{adj})$ is an adjustment factor that can be utilized in a variety of factors to influence the modification of the upper bound. The proportion (1-X and $X$) may be initially set by the automatic drug delivery algorithm as an 80/20 split of maximal limit, for example, $X$ may equal 0.2 to provide the 80/20 split of $UB_{h-1}$ and $UB_h$. The proportion may be adjusted to control the speed of upper bound changes. For example, a proportion of 70/30 would make the upper bound converge to the new value in 5 days, whereas an 80/20 proportion would converge in 7 days.

In one embodiment, the automatic drug delivery algorithm may adjust the upper bound over time based on a user's estimated HbAlc levels to optimize the user's condition. $F_{adj}$ can be defined by an estimated HbA1c, as:

$$f\left(F_{adj,HbA1c_{est}}\right) = UB_{orig} \cdot \max\left(\frac{HbA1c_{est}}{6.4}, 1\right)$$

$$HbA1c_{est} = \frac{\frac{\sum_{i=1}^{n} CGM(n)}{n} + 77.3}{35.6}$$

where 77.3 and 35.6 are fixed constants (conversion factors between HbA1c[%] and mg/dL) and $HbA1c_{est}$ is an estimate of an HbAlc value (which is a percentage). In an example, a value of 6.4 may be the lower bound of HbAlc before a person is considered to have diabetes, and the exemplary embodiment does not decrease the upper bound below the original upper bound $UB_{orig}$.

Alternately, the upper bound may be adjusted by the user's total insulin needs and weight, such as the following:

$$f\left(F_{adj,TDI}\right) = UB_{orig} \cdot \max\left(\frac{TDI_{est}}{0.8 \cdot W_{kg}}, 1\right)$$

$$TDI_{est} = \frac{\sum_{i=1}^{n_{datapoints}} I(i)}{n} \cdot 244$$

Here, 0.8 times the user's weight in TDI may be the typical estimated insulin needs per weight. Therefore, if the user requires significantly increased daily insulin, one embodiment of the calculation of which is described in the equation for $TDI_{est}$ above, then the upper bound of maximum closed loop insulin delivery can be increased.

In an example, process 200 may determine a time for delivery of insulin or any other drug in liquid form to the user. For example, a controller may determine the dosage of the drug at a particular time based on the selected multiple. The particular time may be based on the selected multiple, where if the multiple is large, the delivery may occur earlier to account for the rate of delivery of insulin or the drug.

In block 208, the controller or processor executing programming code to implement the process 200 may determine an amount of a dose of the liquid drug to be delivered based on the calculated upper boundary constraint.

In block 210, process 200 generates a control signal based on the determined amount of the dose of the liquid drug. In block 212, process 200 causes the controller to output the control signal to cause a pump mechanism to expel the dose of the liquid drug from a container.

The controller may execute the process 100 or process 200 multiple times a day or may have a preset time, such as 12 AM, or a preset event, such as when a user sleeping or at set up of a drug delivery device, at which the controller executes the respective process 100 or 200.

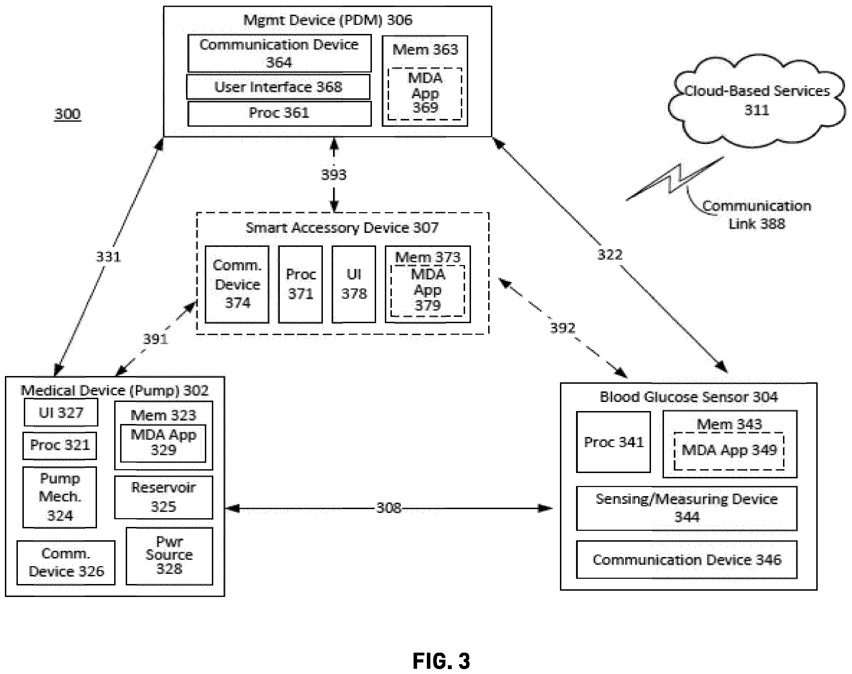

It may be helpful to discuss an example of a drug delivery system that may implement the process examples of FIGs. 1 and 2. FIG. 3 illustrates an example of a drug delivery system 300 suitable for executing the processes 100 or 200.

The drug delivery system 300 may be operable to implement the process examples illustrated in FIGs. 1 and 2 by executing a medication delivery application or algorithm (MDA) that provides functionality, for example, to receive an indication of a type of diabetes that will be controlled by a processor or controller. The processor or controller may obtain a glucose control metric by, for example, retrieving the glucose control metric from a memory, obtaining it from another device in the system (such as a management device 306, a smart accessory device 307 or cloud-based services 311), or calculating it using other information, such as a blood glucose measurement value (obtained, for example, from the analyte sensor 304). The processor or controller may ascertain, based on the indication of the type of diabetes, the glucose control metric or both, an upper boundary constraint for the liquid drug. The upper boundary constraint may be a multiple of a total daily dosage setting for the liquid drug. A time for delivery of the liquid drug to the user may be determined. Prior to the determined time, an amount of a dose of the liquid drug to be delivered based on the upper boundary constraint may be determined by the processor or controller. A control signal may be generated based by the processor or controller on the determined amount of the dose of the liquid drug. The control signal may be applied to the pump mechanism to expel the dose of the liquid drug from the container. As described below, the respective processors or controllers of the drug delivery system 300 may be configured to implement the functions described with reference to FIGs. 1 and 2.

The drug delivery system 300 may be an automated drug delivery system that may include a medical device (pump) 302 (also referred to as "a drug delivery device" or "a wearable drug delivery device"), a analyte sensor 304 (such as a blood glucose sensor, also referred to as "a continuous glucose monitor" or "a blood glucose measurement device"), and a management device (PDM) 306. The drug delivery system 300, in an example, may also include a smart accessory device 307, which may be operable to communicate with the other components of system 300 either via a wired or wireless communication link, such as 391, 392 or 393. The management device 306, medical device 302 and analyte sensor 304 may be operable to communicate via a wired or wireless communication link, such as 331, 322 or 308.

In an example, the medical device 302 may be attached to the body of a user, such as a patient or diabetic, and may deliver any therapeutic agent, including any drug or medicine, such as insulin, morphine or the like, to the user. The medical device 302 may, for example, be a wearable device worn by the user. For example, the medical device 302 may be directly coupled to a user (e.g., directly attached to a body part and/or skin of the user via an adhesive or the like). In an example, a surface of the medical device 302 may include an adhesive (not shown) to facilitate attachment to a user.

The medical device 302 may include a number of components to enable automated delivery of a drug (also referred to as a therapeutic agent) to the user. The medical device 302 may be operable to store the drug (e.g., insulin, glucagon, pain relief drugs, hormones, blood pressure medicines, morphine, methadone, chemotherapy drugs, or the like) and to provide the drug to the user. The medical device 302 may also be referred to as a "pump", "pump mechanism," "mech." or "an insulin pump," in reference to the operation of expelling insulin from the reservoir 325 for delivery to the user. While the examples refer to the reservoir 325 storing insulin, the reservoir 325 may be operable to store other drugs or therapeutic agents, such as those listed above or the like, for automated delivery.

In various examples, the medical device 302 may be an automated, wearable drug delivery device. As shown in FIG. 3, the medical device 302 may include a reservoir 325 for storing the drug, a needle or cannula (not shown) for delivering the drug into the body of the user (which may be done subcutaneously, intraperitoneally, or intravenously), and a pump mechanism (mech.) 324, or other drive mechanism, for transferring the drug from the reservoir 325, through a needle or cannula (not shown), and to the user. The pump mechanism 324 may be fluidly coupled to reservoir 325, and communicatively coupled to the medical device controller 321. The medical device 302 may also include a power source 328, such as a battery, a piezoelectric device, or the like, for supplying electrical power to the pump mechanism 324 and/or other components (such as the controller 321, memory 323, user interface 327, and the communication device 326) of the medical device 302. Although not shown, a power source for supplying electrical power may similarly be included in each of the sensor 304, the smart accessory device 307 and the management device (PDM) 306.

The medical device 302 may contain analog and/or digital circuitry that may be implemented as a controller 321 (or processor) for controlling the delivery of the drug or therapeutic agent. The circuitry used to implement the controller 321 may include discrete, specialized logic and/or components, an application-specific integrated circuit, a microcontroller or processor that executes software instructions, firmware, programming instructions or programming code (enabling, for example, execution of programming code to implement functions of the artificial pancreas application (MDA APP) 329 as well as the process examples of FIGs. 1 and 2) stored in memory 323, or any combination thereof. For example, the controller 321 may execute a control algorithm, such as an artificial pancreas application 329, and other programming code that may make the controller 321 operable to cause the pump to deliver doses of the drug or therapeutic agent to a user at predetermined intervals or as needed to bring blood glucose measurement values to a target blood glucose value. In an example, the MDA application (MDA APP) 329 may include programming code that is operable upon execution by the controller 321 to provide the example processes that estimate a glucose control metric and base a calculation of an upper boundary constraint for a liquid drug on the estimated glucose control metric, or the like as described with reference to FIGs. 1 and 2. A table related to the blood glucose metric may be programmed, for example, into an artificial pancreas application 329 by the user or by a third party (such as a health care provider, medical device manufacturer, or the like) using a wired or wireless link, such as 331, between the medical device 302 and a management device 306 or other device, such as a computing device at a healthcare provider facility. In an example, the pump or medical device 302 is communicatively coupled to the processor 361 of the management device via the wireless link 331 or via a wireless link, such as 391 from smart accessory device 307 or 308 from the sensor 304. The pump mechanism 324 of the medical device 302 may be operable to receive a control signal from the processor 361, and in response to receiving the control signal, may expel insulin from the reservoir 325 based on the evaluations, calculations, determinations, and process steps performed in the process examples of FIGs. 1 and 2.

Instructions for determining the delivery of the drug or therapeutic agent (e.g., as a bolus dosage) to the user (e.g., the size and/or timing of any doses of the drug or therapeutic agent) may originate locally by the medical device 302 or may originate remotely and be provided to the medical device 302. In an example of a local determination of drug or therapeutic agent delivery, programming instructions, such as an instance of the artificial pancreas application 329, stored in the memory 323 that is coupled to the medical device 302 may be used to make determinations by the medical device 302. In addition, the medical device 302 may be operable to communicate with the cloud-based services 311 via the communication device 326 and the communication link 388.

Alternatively, the remote instructions may be provided to the medical device 302 over a wired or wireless link (such as 331) by the management device (PDM) 306, which has a processor 361 that executes an instance of the artificial pancreas application 369, or the smart accessory device 307 (via communication link 391), which has a processor 371 that executes an instance of the artificial pancreas application 369 as well as other programming code for controlling various devices, such as the medical device 302, smart accessory device 307 and/or sensor 304. The medical device 302 may execute any received instructions (originating internally or from the management device 306) for the delivery of the drug or therapeutic agent to the user. In this way, the delivery of the drug or therapeutic agent to a user may be automated.

In various examples, the medical device 302 may communicate via a wireless link 331 with the management device 306. The management device 306 may be an electronic device such as, for example, a smart phone, a tablet, a dedicated diabetes therapy management device, or the like. The management device 306 may be a wearable wireless accessory device. The wireless links 308, 331, 322, 391, 392 and 393 may be any type of wireless link provided by any known wireless standard. As an example, the wireless links 308, 331, 322, 391, 392 and 393 may enable communications between the medical device 302, the management device 306 and sensor 304 based on, for example, Bluetooth®, Wi-Fi®, a near-field communication standard, a cellular standard, or any other wireless optical or radio-frequency protocol.

In the drug delivery system 300, the analyte sensor 304 may be a device communicatively coupled to the processor 361 or 321 and may be operable to measure a blood glucose value at a predetermined time interval, such as every 5 minutes, or the like. The analyte sensor 304 may provide a number of blood glucose measurement values to one or more of the MDA applications (e.g., 329, 349, 369, or 379) operating on the respective devices, 302, 304, 306 and 307. In addition, the analyte sensor 304 may be operable to detect and provide measurement values related to other physical attributes or analytes, such as hormone levels, heart rate, blood oxygen levels, ketones, lactates, uric acid, sodium, potassium, alcohol, proteins, hormones, or the like.

The sensor 304 may be a glucose sensor operable to measure blood glucose and output a blood glucose value or data that is representative of a blood glucose value. For example, the sensor 304 may be a glucose monitor or a continuous glucose monitor (CGM). The sensor 304 may include a processor 341, a memory 343, a sensing/measuring device 344, and communication device 346. The communication device 346 of sensor 304 may include one or more of a transmitter, a receiver, and/or a transceiver for communicating with the management device 306 over a wireless link 322 or with medical device 302 over the link 308. The sensing/measuring device 344 may include one or more sensing elements, such as a glucose measurement, heart rate monitor, an analyte sensor, a hormone detector, blood pressure monitor, or the like. The processor 341 may include discrete, specialized logic and/or components, an application-specific integrated circuit, a microcontroller or processor that executes software instructions, firmware, programming instructions stored in memory (such as memory 343), or any combination thereof. For example, the memory 343 be a random access memory or the like and may store an instance of an MDA application 349 that is executable by the processor 341.

Although the sensor 304 is depicted as separate from the medical device 302, in various examples, the sensor 304 and medical device 302 may be incorporated into the same unit. That is, in various examples, the sensor 304 may be a part of the medical device 302 and contained within the same housing as the medical device 302 (e.g., the sensor 304 may be positioned within or embedded within the medical device 302). Glucose monitoring data (e.g., measured blood glucose values) determined by the sensor 304 may be provided to the medical device 302, smart accessory device 307 and/or the management device 306 via wired or wireless communication links 308, 392 or 322. The sensor 304 may also be coupled to the user by, for example, adhesive or the like.

In an operational example, the MDA application 369 may be executing in the management device 306 and control delivery of insulin by sending control signals or command signals to the medical device 302. The management device memory 363 may store an instance of the MDA application 369 that includes programming code, that when executed by the processor 361 provides the process examples described with reference to the examples of FIGs. 1 and 2. For example, the MDA application 369 may be operable to perform the functions described with reference to the processes 100 and 200 described with reference to FIGs. 1 and 2. The processor 361 executing the MDA application 369 may determine the timing of an insulin dose and may output a command signal to the medical device 302 that actuates the pump mechanism 324 to deliver insulin dose based on the evaluations and process steps performed in the process examples of FIGs. 1 and 2.

The other devices in the system 300, such as management device 306, smart accessory device 307 and sensor 304, may also be operable to perform various functions including controlling the medical device 302. For example, the management device 306 may include a communication device 364, a processor 361, and a management device memory 363. The management device memory 363 may also store programming code for providing the process examples described with reference to the examples of FIGs. 1 and 2.

The smart accessory device 307 may be, for example, an Apple Watch®, other wearable smart device, including eyeglasses, provided by other manufacturers, a global positioning system-enabled wearable, a wearable fitness device, smart clothing, or the like. Like the management device 306, the smart accessory device 307 may also be operable to perform various functions including controlling the medical device 302. For example, the smart accessory device 307 may include a communication device 374, a processor 371, and a memory 373. The memory 373 may store an instance of the MDA application 379 that includes programming code for providing the process examples described with reference to the examples of FIGs. 1 and 2. The memory 373 may also as store programming code and be operable to store data related to the MDA application 379. The sensor 304 of system 300 may be a continuous glucose monitor (CGM) as described above, that may include a processor 341, a memory 343, a sensing or measuring device 344, and a communication device 346. The memory 343 may, for example, store an instance of an MDA application 349 as well as other programming code and be operable to store data related to the MDA application 349 and process examples described with reference to FIGs. 1 and 2. The MDA application 349 may also include programming code for providing the process examples described with reference to the examples of FIGs. 1 and 2.

In the drug delivery device system 300, the management device 306 may be a computing device operable to manage a personal diabetes treatment plan via an MDA application or an algorithm. The management device 306 may be used to program or adjust operation of the medical device 302 and/or the sensor 304. For example, the management device 306 may be configured to initially set up a medical device 302. The management device 306 may be any portable electronic, computing device including, for example, a dedicated controller, such as processor 361, a smartphone, or a tablet. In an example, the management device (PDM) 306 may include a processor 361, a management device management device memory 363, and a communication device 364. The management device 306 may contain analog and/or digital circuitry that may be implemented as a processor 361 (or controller) for executing processes to manage a user's blood glucose levels and for controlling the delivery of the drug or therapeutic agent to the user. The processor 361 may also be operable to execute programming code stored in the management device management device memory 363. For example, the management device management device memory 363 may be operable to store an artificial pancreas (AP) application 369 that may be executed by the processor 361. The processor 361 may when executing the artificial pancreas application 369 may be operable to perform various functions, such as those described with respect to the examples in FIGs. 1 and 2. The communication device 364 may be a receiver, a transmitter, or a transceiver that operates according to one or more radio-frequency protocols. For example, the communication device 364 may include a cellular transceiver and a Bluetooth transceiver that enables the management device 306 to communicate with a data network via the cellular transceiver and with the sensor 304 and the medical device 302. The respective transceivers of communication device 364 may be operable to transmit signals containing information useable by or generated by the MDA application or the like. The communication devices 326, 346 and 376 of respective medical device 302, sensor 304 and smart accessory device 307 may also be operable to transmit signals containing information useable by or generated by the MDA application or the like.

The medical device 302 may communicate with the sensor 304 over a wireless link 308 and may communicate with the management device 306 over a wireless link 331. The sensor 304 and the management device 306 may communicate over a wireless link 322. The smart accessory device 307, when present, may communicate with the medical device 302, the sensor 304 and the management device 306 over wireless links 391, 392 and 393, respectively. The wireless links 308, 331, 322, 391, 392 and 393 may be any type of wireless link operating using known wireless standards or proprietary standards. As an example, the wireless links 308, 331, 322, 391, 392 and 393 may provide communication links based on Bluetooth®, Wi-Fi, a near-field communication standard, a cellular standard, or any other wireless protocol via the respective communication devices 326, 346 and 364. In some examples, the medical device 302 and/or the management device 306 may include a user interface 327, 378 and 368, respectively, such as a keypad, a touchscreen display, levers, buttons, a microphone, a speaker, a display, or the like, that is operable to allow a user to enter information and allow the management device to output information for presentation to the user.

In various examples, the drug delivery system 300 may implement the artificial pancreas (AP) algorithm (and/or provide AP functionality) to govern or control automated delivery of insulin to a user (e.g., to maintain euglycemia - a normal level of glucose in the blood). The MDA application may be implemented by the medical device 302 and/or the sensor 304. The MDA application may be used to implement the process 100 of FIG. 1 or the process 200 of FIG. 3. The MDA application may also allow the user to adjust insulin delivery. For example, the MDA application may allow the user to issue (e.g., via an input) commands to the medical device 302, such as a command to deliver an insulin bolus. In some examples, different functions of the MDA application may be distributed among two or more of the management device 306, the medical device (pump) 302 or the sensor 304. In other examples, the different functions of the MDA application may be performed by one device, such the management device 306, the medical device (pump) 302 or the sensor 304.

As described herein, the drug delivery system 300 or any component thereof, such as the medical device 302 may provide AP functionality or implement an MDA application. Accordingly, references to the MDA application (e.g., functionality, operations, or capabilities thereof) are made for convenience and may refer to and/or include operations and/or functionalities of the drug delivery system 300 or any constituent component thereof (e.g., the medical device 302 and/or the management device 306). The drug delivery system 300 - for example, as an insulin delivery system implementing an MDA application - may be considered to be a drug delivery system or an MDA application-based delivery system that uses sensor inputs (e.g., data collected by the sensor 304).

In an example, one or more of the devices, 302, 304, 306 or 307 may be operable to communicate via a wireless communication link 388 with cloud-based services 311. The cloud-based services 311 may utilize servers and data storage (not shown). The communication link 388 may be a cellular link, a Wi-Fi link, a Bluetooth link, or a combination thereof, that is established between the respective devices 302, 304, 306 or 307 of system 300. The data storage provided by the cloud-based services 311 may store anonymized data, such as user weight, blood glucose measurements, age, meal carbohydrate information, or the like. In addition, the cloud-based services 311 may process the anonymized data from multiple users to provide generalized information related to the various parameters used by the MDA application. For example, an age-based general target blood glucose value may be derived from the anonymized data, which may be helpful when a user first begins using a system such as 300. The cloud-based services 311 may also provide processing services for the system 300, such as performing the process 100 in the example of FIG. 2 or additional processes, such as that described below with reference to FIG. 3.

In an example, the device 302 includes a communication device 364, which as described above may be a receiver, a transmitter, or a transceiver that operates according to one or more radio-frequency protocols, such as Bluetooth, Wi-Fi, a near-field communication standard, a cellular standard, that may enable the respective device to communicate with the cloud-based services 311. For example, outputs from the sensor 304 or the medical device (pump) 302 may be transmitted to the cloud-based services 311 for storage or processing via the transceivers of communication device 364. Similarly, medical device 302, management device 306 and sensor 304 may be operable to communicate with the cloud-based services 311 via the communication link 388.

In an example, the respective receiver or transceiver of each respective device, 302, 306 or 307, may be operable to receive signals containing respective blood glucose measurement values of the number of blood glucose measurement values that may be transmitted by the sensor 304. The respective processor of each respective device 302, 306 or 307 may be operable to store each of the respective blood glucose measurement values in a respective memory, such as 323, 363 or 373. The respective blood glucose measurement values may be stored as data related to an artificial pancreas algorithm, such as MDA APPs 329, 349, 369 or 379. In a further example, the MDA application operating on any of the management device 306, the smart accessory device 307, or sensor 304 may be operable to transmit, via a transceiver implemented by a respective communication device, 364, 374, 346, a control signal for receipt by a medical device. In the example, the control signal may indicate an amount of insulin to be expelled by the medical device 302.

Various operational scenarios and examples of processes performed by the system 300 are described herein. For example, the system 300 may be operable to implement the process examples of FIG. 1 and 2.

The techniques described herein for providing functionality to determine upper boundary constraints to limit an amount of a drug to be delivered to users with different types of diseases, such as T1DM and T2DM, and deliver a determined amount of the drug to the respective users. For example, the system 300 or any component thereof may be implemented in hardware, software, or any combination thereof. Software related implementations of the techniques described herein may include, but are not limited to, firmware, application specific software, or any other type of computer readable instructions that may be executed by one or more processors. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system, such as system 300, or constituent component described herein may be implemented with a processor executing computer readable instructions stored on one or more memory components.

FIG. 4 illustrates upper bound examples 400 according to the examples described herein. The fixed clinical multiple setting 402 may be a setting for an upper boundary of a user diagnosed as being a T1DM diabetic. The increased upper bound 404 may apply to a user diagnosed as a T2DM diabetic, whose condition causes wide fluctuations in insulin usage and efficacy. As a result, the T2DM diabetic is better able to handle and benefit from the increased upper bound 404.

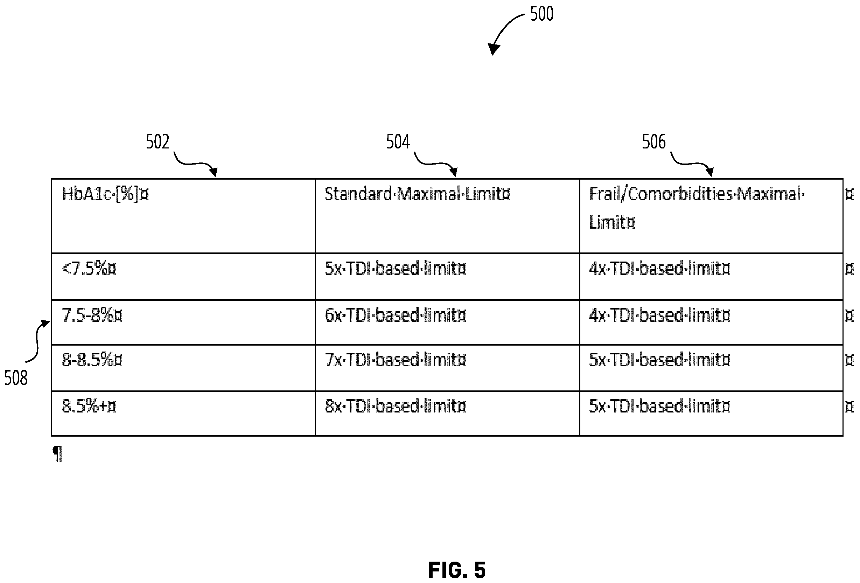

FIG. 5 illustrates an example of data structure suitable for use with the disclosed examples. As shown in the example of FIG. 5, a data structure 500, shown as a table, may have a glucose control metric 502, a standard maximal limit 504 and a frail/comorbidities maximal limit 506. The glucose control metric 502 may be based on different physical attributes of a user, such as HbAlc [%], blood glucose measurements, or the like. The determination of the standard maximal limit 504 may be determined based on a correlation of the glucose control metric 502 (i.e., HbAlc %) to Standard Maximal Limit 504. The glucose control metric 502 may be a set of ranges of percentages, such as < 7.5%, 7.5%-8.0%, 8-8.5% and 8.5% +. Onset of diabetes is commonly diagnosed as occurring when HbA1C % is $\geq$ 6.5%. The standard maximal limit 504 may correlate to a respective set of the ranges of the glucose control metric 502 as the user's HbAlC percentage increases as the user's diabetes becomes more acute (i.e., the user's diabetic condition worsens) or improves (e.g., the HbA1C percentage decreases). As shown in FIG. 5, as the user's glucose control metric 502 increases, the standard maximal limit 504 also increases. However, when a user has a comorbidity or a frailty, the MDA application may determine that the standard maximal limit 504 is not appropriate and may default the upper boundary setting to frail/comorbidities maximal limit 506. For example, a user may have a HbAlC % of 7.7, the MDA application may determine that an upper boundary setting should be equal to 6 times (6x) the user's total daily insulin (TDI) based basal limit as shown a row 508. However, if the user has or develops a comorbidity, such as asthma or obesity, the MDA application may determine that the upper boundary setting should be 4 times (4x) the user's total daily insulin (TDI) based basal limit (also shown in row 508).

Software related implementations of the techniques described herein, such as the processes examples described with reference to FIGs. 1-3 may include, but are not limited to, firmware, application specific software, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

In addition, or alternatively, while the examples may have been described with reference to a closed loop algorithmic implementation, variations of the disclosed examples may be implemented to enable open loop use. The open loop implementations allow for use of different modalities of delivery of insulin such as smart pen, syringe or the like. Blood glucose measurements may be provided for open-loop input from a blood glucose monitor, a continuous glucose monitor, or the like. A management device may maintain the data history and adjust or recommend system settings. For example, the disclosed MDA application and algorithms may be operable to perform various functions related to open loop operations, such as the generation of prompts requesting the input of information such as weight or age. Similarly, a dosage amount of insulin may be received by the MDA application or algorithm from a user via a user interface. Other open-loop actions may also be implemented by adjusting user settings or the like in an MDA application or algorithm.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, subject matter that lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

## Claims

1. A wearable drug delivery device, comprising:

   a controller;
   a container containing a liquid drug;
   a pump mechanism coupled to the controller and coupled to the pump mechanism, wherein the pump mechanism is configured to expel the liquid drug from the container in response to control signals from the controller; and

a memory storing an automatic drug delivery algorithm, the automatic drug delivery algorithm when executed by the controller, configures the controller to:

receive an indication of a type of diabetes that will be controlled by the controller;
receive a glucose control metric;
ascertain, based on the indication of the type of diabetes and the glucose control metric, an upper boundary constraint for the liquid drug, wherein the upper boundary constraint is a multiple of a total daily dosage setting for the liquid drug;
determine an amount of a dose of the liquid drug to be delivered based on the upper boundary constraint;
generate a control signal based on the determined amount of the dose of the liquid drug; and
apply the control signal to the pump mechanism to expel the amount of the dose of the liquid drug from the container.

2. The wearable drug delivery device of claim 1, wherein the controller is further configured by execution of the automatic drug delivery algorithm to:

receive an indication of a comorbidity of a user; and
adjust, based on the indication of the comorbidity, the upper boundary constraint for the liquid drug; and
use the adjusted upper boundary constraint to determine the amount of the dose of the liquid drug to be delivered based on the upper boundary constraint.

3. The wearable drug delivery device of claim 1, wherein the controller is further configured by execution of the automatic drug delivery algorithm to:

based on the indication of the type of diabetes, evaluate the glucose control metric to determine a setting for the multiple of the total daily dosage setting; and
store the glucose control metric, the determined multiple in the memory and the upper boundary constraint in the memory.

4. The wearable drug delivery device of claim 1, wherein the controller is further configured by execution of the automatic drug delivery algorithm to:

in response to the indication of the type of diabetes being for Type 1 diabetes mellitus, set the multiple of the total daily dosage setting to a fixed clinical multiple setting; and
store the glucose control metric, the fixed clinical multiple setting in the memory and the upper boundary constraint in the memory.

5. The wearable drug delivery device of claim 1, wherein the controller is further configured by execution of the automatic drug delivery algorithm to:

in response to the indication of the type of diabetes being for Type 2 diabetes mellitus, evaluate the glucose control metric to determine the multiple of the total daily dosage setting; and
store the glucose control metric, the determined multiple in the memory and the upper boundary constraint in the memory.

6. The wearable drug delivery device of claim 5, wherein, when evaluating the glucose control metric to determine the multiple of the total daily dosage setting, the controller is further configured by execution of the automatic drug delivery algorithm to:
in response to the indication of the type of diabetes being for Type 2 diabetes mellitus, select a multiple from a list of multiples of the total daily dosage setting, wherein the selected multiple corresponds to the glucose control metric found in the list, wherein the multiple increases based on a range of the glucose control metric.

7. The wearable drug delivery device of claim 1, wherein the glucose control metric is a percentage of red blood cells that have sugar-coated hemoglobin over a period of time.

8. The wearable drug delivery device of claim 1, further comprising:
a communication interface coupled to the controller, the communication interface including circuitry configured to:

respond to wireless signals or haptic inputs,
receive the glucose control metric via a wireless signal or a haptic input, and
forward the glucose control metric to the controller.

9. A drug delivery system, comprising:

a processor; and
a memory coupled to the processor and storing an automatic drug delivery algorithm, wherein the automatic drug delivery algorithm when executed by the processor, configures the processor to:

determine an average blood glucose measurement value over a period of time;
determine an estimated glucose control metric using the average blood glucose measurement value;
calculate, based on the estimated glucose control metric, an upper boundary constraint for a liquid drug;
determine an amount of a dose of the liquid drug to be delivered based on the calculated upper boundary constraint;
generate a control signal based on the determined amount of the dose of the liquid drug; and
output the control signal to cause a pump mechanism to expel the dose of the liquid drug from a container.

10. The drug delivery system of claim 9, wherein the calculated upper boundary constraint is calculated based on a previous upper boundary constraint and an interim upper boundary constraint for a maximum drug delivery limit for the liquid drug.

11. The drug delivery system of claim 10, wherein the automatic drug delivery algorithm when executed by the processor configures the processor, when calculating an upper boundary constraint for the liquid drug, to:

determine a number of days covered by the period of time;
determine a proportion of the number of days to be attributed to a previous upper boundary constraint and a remainder of the proportion to be attributed to the interim upper boundary constraint;
multiply the previous upper boundary constraint by the proportion of the number of days to be attributed to the previous upper boundary constraint to obtain a first result;
multiply the interim upper boundary constraint by the remainder of the proportion attributed to the interim upper boundary constraint to obtain a second result;
sum the first result and the second result; and
store the sum in the memory as the calculated upper boundary constraint.

12. The drug delivery system of claim 9, further comprising:

a communication interface coupled to the processor, wherein the communication interface includes a transceiver configured to receive wireless communication signals,
wherein the automatic drug delivery algorithm when executed by the processor, configures the processor to:

receive via the communication interface a wireless communication signal with a blood glucose measurement; and
store the received blood glucose measurement in a blood glucose history in the memory.

13. The drug delivery system of claim 9, wherein the automatic drug delivery algorithm when executed by the processor configures the processor, when determining an average blood glucose measurement value over the period of time, to:

retrieve a blood glucose history from the memory,
identify a number of blood glucose measurements over the period of time in the blood glucose history; and
calculate the average blood glucose measurement value using the identified number of blood glucose measurements.

100

RECEIVE AN INDICATION OF A TYPE OF DIABETES THAT WILL BE CONTROLLED BY THE PROCESSOR 102

OBTAIN A GLUCOSE CONTROL METRIC 104

ASCERTAIN, BASED ON THE INDICATION OF THE TYPE OF DIABETES AND THE GLUCOSE CONTROL METRIC, A UPPER BOUNDARY CONSTRAINT FOR THE LIQUID DRUG 106

DETERMINE AN AMOUNT OF A DOSE OF THE LIQUID DRUG TO BE DELIVERED BASED ON THE UPPER BOUNDARY CONSTRAINT 108

GENERATE A CONTROL SIGNAL BASED ON THE DETERMINED AMOUNT OF THE DOSE OF THE LIQUID DRUG 110

APPLY THE CONTROL SIGNAL TO THE PUMP MECHANISM TO EXPEL THE DOSE OF THE LIQUID DRUG FROM THE CONTAINER 112

**FIG. 1**

200

DETERMINE AN AVERAGE BLOOD GLUCOSE MEASUREMENT VALUE OVER A PERIOD OF TIME 202

ESTIMATE AN ESTIMATED GLUCOSE CONTROL METRIC USING THE AVERAGE BLOOD GLUCOSE MEASUREMENT VALUE 204

CALCULATE, BASED ON THE ESTIMATED GLUCOSE CONTROL METRIC, AN UPPER BOUNDARY CONSTRAINT FOR THE LIQUID DRUG 206

DETERMINE AN AMOUNT OF A DOSE OF THE LIQUID DRUG TO BE DELIVERED BASED ON THE CALCULATED UPPER BOUNDARY CONSTRAINT 208

GENERATE A CONTROL SIGNAL BASED ON THE DETERMINED AMOUNT OF THE DOSE OF THE LIQUID DRUG 210

OUTPUT THE CONTROL SIGNAL TO CAUSE A PUMP MECHANISM TO EXPEL THE DOSE OF THE LIQUID DRUG FROM A CONTAINER 212

**FIG. 2**

**FIG. 3**

FIG. 4

| HbA1c [%]¤ | Standard Maximal Limit¤ | Frail/Comorbidities Maximal Limit¤ | ¤ |
|---|---|---|---|
| <7.5%¤ | 5x TDI based limit¤ | 4x TDI based limit¤ | ¤ |
| 7.5-8%¤ | 6x TDI based limit¤ | 4x TDI based limit¤ | ¤ |
| 8-8.5%¤ | 7x TDI based limit¤ | 5x TDI based limit¤ | ¤ |
| 8.5%+¤ | 8x TDI based limit¤ | 5x TDI based limit¤ | ¤ |

**FIG. 5**

EP 4 039 293 A1

Europäisches
Patentamt
European
Patent Office
Office européen
des brevets

# EUROPEAN SEARCH REPORT

Application Number

EP 22 15 5269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/390973 A1 (WU DI [US] ET AL) 17 December 2020 (2020-12-17) * figures 1, 2, 3, 4, 5, 6 * * paragraphs [0018] – [0073] * | 1–13 | INV. A61M5/142 |
| X | US 2020/101225 A1 (O'CONNOR JASON [US] ET AL) 2 April 2020 (2020-04-02) * figures 1, 2, 3, 4, 5–6B * * paragraphs [0016] – [0060], [0065] – [0114] * | 1–13 | |
| A | WO 2017/209903 A1 (ROCHE DIABETES CARE INC [US]; ROCHE DIABETES CARE INC [US] ET AL.) 7 December 2017 (2017-12-07) * the whole document * | 1–13 | |
| A | EP 3 721 921 A1 (MICROTECH MEDICAL HANGZHOU CO LTD [CN]) 14 October 2020 (2020-10-14) * the whole document * | 1–13 | |
| A | US 2018/280619 A1 (FINAN DANIEL [US] ET AL) 4 October 2018 (2018-10-04) * the whole document * | 1–13 | TECHNICAL FIELDS SEARCHED (IPC) A61M |

The present search report has been drawn up for all claims

| Place of search | Date of completion of the search | Examiner |
|---|---|---|
| Munich | 28 June 2022 | Benes, Václav |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 5269

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2022

| Patent document cited in search report | | Publication date | Patent family member(s) | | Publication date |
|---|---|---|---|---|---|
| US 2020390973 | A1 | 17-12-2020 | NONE | | |
| US 2020101225 | A1 | 02-04-2020 | AU | 2019347755 A1 | 13-05-2021 |
| | | | CA | 3112209 A1 | 02-04-2020 |
| | | | CN | 112789070 A | 11-05-2021 |
| | | | EP | 3856285 A1 | 04-08-2021 |
| | | | JP | 2022501139 A | 06-01-2022 |
| | | | US | 2020101225 A1 | 02-04-2020 |
| | | | WO | 2020069406 A1 | 02-04-2020 |
| WO 2017209903 | A1 | 07-12-2017 | AU | 2017275393 A1 | 03-01-2019 |
| | | | BR | 112018074909 A2 | 12-03-2019 |
| | | | CA | 3025902 A1 | 07-12-2017 |
| | | | CN | 109478422 A | 15-03-2019 |
| | | | EP | 3479262 A1 | 08-05-2019 |
| | | | KR | 20190013922 A | 11-02-2019 |
| | | | US | 2017348482 A1 | 07-12-2017 |
| | | | WO | 2017209903 A1 | 07-12-2017 |
| EP 3721921 | A1 | 14-10-2020 | CN | 107715230 A | 23-02-2018 |
| | | | EP | 3721921 A1 | 14-10-2020 |
| | | | US | 2021187195 A1 | 24-06-2021 |
| | | | WO | 2019072141 A1 | 18-04-2019 |
| US 2018280619 | A1 | 04-10-2018 | AU | 2018243290 A1 | 24-10-2019 |
| | | | BR | 112019020316 A2 | 09-06-2020 |
| | | | CA | 3058440 A1 | 04-10-2018 |
| | | | CN | 110868927 A | 06-03-2020 |
| | | | EP | 3600037 A1 | 05-02-2020 |
| | | | JP | 2020512153 A | 23-04-2020 |
| | | | KR | 20190137844 A | 11-12-2019 |
| | | | RU | 2019134803 A | 30-04-2021 |
| | | | TW | 201901604 A | 01-01-2019 |
| | | | US | 2018280619 A1 | 04-10-2018 |
| | | | WO | 2018183689 A1 | 04-10-2018 |

EPO FORM P0459

For more details about this annex : see Official Journal of the European Patent Office, No. 12/82